# EUROPEAN PATENT APPLICATION

(11) **EP 4 458 302 A1**
(43) Date of publication of application: **06.11.2024**
(21) Application number: 22914824.2
(22) Date of filing: 27.12.2022
(51) Int. Cl.: A61B 18/14

(54) **ADAPTIVE GUIDANCE DEVICE AND TRANSCATHETER TREATMENT SYSTEM**

(30) Priority: 31.12.2021 CN 202111679305
(71) Applicant: Hangzhou Deke Medtech Co., Ltd., Hangzhou, Zhejiang 310052 (CN)
(72) Inventor: SHAO, Nan, Hangzhou Zhejiang 310052 (CN); LI, Yang, Hangzhou Zhejiang 310052 (CN); ZHUANG, Zhenping, Hangzhou Zhejiang 310052 (CN); ZENG, Jianfeng, Hangzhou Zhejiang 310052 (CN); GAN, Yijie, Hangzhou Zhejiang 310052 (CN)
(74) Representative: Zaboliene, Reda
(86) International application number: PCT/CN2022/142506
(87) International publication number: WO 2023/125572

(57) **Abstract**

The present disclosure discloses an adaptive guiding device and a transcatheter treatment system. The adaptive guiding device includes a hollow flexible catheter, which is pre-shaped and has a bend in a natural state. The distal part of the flexible catheter includes a distal section, the distal section includes a transition segment and a straight segment, the transition segment is bendable so as to change an orientation of the straight segment, and a bending force application point is adjacent to a connection of the transition segment with the straight segment. In the adaptive guiding device and the transcatheter treatment system provided by the present disclosure, the adaptability of the guiding device to blood vessels with complex anatomical shapes such as the aortic arch is improved, the structure of the guiding device is simplified, and the operation safety of the device is improved. There is no need for a large avoidance space in use of the guiding device, and the tissue can be prevented from extending into the avoidance area, so that the guiding device has a larger operation and adjustment space and a more convenient initial position for adjustment.

## Description

### TECHNICAL FIELD

The present disclosure relates to the technical field of medical devices, and in particular to an adaptive guiding device and a transcatheter treatment system.

### DESCRIPTION OF THE PRIOR ART

With the development of medical technology, interventional surgery is becoming more and more common, which is characterized by opening a small operating window on the patient's body surface, delivering devices such as catheter through the vascular access to the treatment site to establish a passage from outside the body to inside the body, and then delivering various treatment devices through this passage for interventional treatment. Among them, the device used to establish a passage from outside the body to inside the body is the so-called guiding device. For interventional treatments through different paths, the guiding device needs to adapt to the morphologies of different blood vessels, and is preferred to have a certain degree of adaptability to avoid the risk of irreversibly damaging the blood vessel tissue and reach the diseased area quickly and accurately for interventional treatment.

For example, in the prior art, patent application No. WO2020/068601A1 discloses a guiding device for catheter-type interventional device, which uses a distal flexible segment as a hinge structure to cause lateral displacement by applying a certain axial force, to adapt to the curved blood vessel shape.

In the field of interventional therapy of structural heart diseases, conventional interventional paths include: puncture via the femoral vein, through the inferior vena cava, right atrium to the right ventricle, or through the inferior vena cava, right atrium, left atrium to the left ventricle; or puncture via the femoral artery, through the aortic arch to the left ventricle. For the interventional path through the aortic arch, due to the special arc-shaped stereoscopic morphology of the aortic arch, the guiding device is required to include multiple bendable sections, i.e., multiple sections hinged in sequence and cooperated with each other, which will increase the structural complexity and reduce the stability. Further, the hinge structure, when being displaced laterally, requires large avoidance spaces, and the avoidance spaces will be gradually reduced after the lateral displacement, increasing the bending difficulty. In addition, because the vascular tissue is flexible with strong compliance, the tissue may extend into the avoidance area, causing irreversible damage to the vascular tissue and increasing the operation risk of the treatment device.

Therefore, it is necessary to propose a guiding device with high safety and an application plan therefor.

### SUMMARY OF THE DISCLOSURE

In order to overcome at least one of the above-mentioned defects in the prior art, the present disclosure discloses an adaptive guiding device to solve the problems of the existing guiding devices of poor adaptability and easy damage to the human body.

Another object of the present disclosure is to provide a transcatheter treatment system to solve the problems of the existing transcatheter treatment systems of poor adaptability and easy damage to the human body.

The adaptive guiding device includes a hollow flexible catheter, which is pre-shaped and has a bend in a natural state.

A distal part of the flexible catheter includes a distal section, the distal section includes a transition segment and a straight segment, the transition segment is bendable so as to change an orientation of the straight segment, and a bending force application point is adjacent to a connection of the transition segment with the straight segment.

Optionally, the straight segment has a length I, where 0 < I < 80 mm.

Optionally, the length of the straight segment is in a range of 40 to 80 mm.

Optionally, the transition segment is pre-shaped into an arc shape.

Optionally, the transition segment has a central angle a5, and an arc bend of the transition segment has a radius R5, where 15° ≤ a5 ≤ 70°, and 25 mm ≤ R5 ≤ 45 mm.

Optionally, the flexible catheter includes a main section, a trans-aortic arch section and the distal section connected in sequence from proximal to distal and communicated with each other, and wherein the trans-aortic arch section has a curved structure simulating an aortic arch shape, and two ends of the transition segment are respectively tangent to the straight segment and the trans-aortic arch section.

Optionally, the flexible catheter in the natural state is bent multiple times along its central axis toward different planes.

Optionally, the flexible catheter is bent at least five times.

Optionally, angles between planes of at least four consecutive bends are less than 90°.

Optionally, an angle between two planes of one bend closest to a distal end is greater than 90°.

Optionally, the trans-aortic arch section includes a first arc segment, a second arc segment, a third arc segment and a fourth arc segment that are sequentially connected and tangent one after another, the first arc segment, the second arc segment, the third arc segment and the fourth arc segment are distributed on different planes, and wherein the first arc segment is connected and communicated with the main section, and the fourth arc segment is connected and communicated with the distal section.

Optionally, the first arc segment has an angle a1, an arc bend of the first arc segment has a radius R1; the second arc segment has an angle a2, an arc bend of the second arc segment has a radius R2; the third arc segment has an angle a3, the arc bend of the third arc segment has a radius R3; and the fourth arc segment has an angle a4, and an arc bend of the fourth arc segment has a radius R4.

4.5° ≤ a1 ≤ 30°, 90 mm ≤ R1 ≤ 450 mm; 25° ≤ a2 ≤ 80°, 10 mm ≤ R2 ≤ 60 mm; 20° ≤ a3 ≤ 70°, 25 mm ≤ R3 ≤ 50 mm; 20° ≤ a4 ≤ 110 °, 20 mm ≤ R4 ≤ 50 mm.

Optionally, angles between a central axis of a projection of the straight segment on a first plane, a second plane, and a third plane, respectively, and a central axis of a projection of the main section are α, β, and γ respectively, wherein the first plane is a vertical plane, the second plane is perpendicular plane perpendicular to the vertical plane, and the third plane is a horizontal plane perpendicular to both the vertical plane and the perpendicular plane, where 35° ≤ α ≤ 85°, 25° ≤ β ≤ 80°, and 20° ≤ γ ≤ 75°.

Optionally, an angle between a central axis of a projection of the transition segment on the first plane and the central axis of the main section is δ, where 0 < δ ≤ 45°.

Optionally, the adaptive guiding device further includes an operating handle, and the operating handle is connected to the main section and is configured to drive the flexible catheter to move axially and circumferentially.

Optionally, the adaptive guiding device further includes a pull assembly, wherein the pull assembly includes a pull wire, a guiding passageway and an anchor ring, the guiding passageway is defined in a wall of the flexible catheter for the pull wire to pass through, the anchor ring is fixedly connected to the transition segment of the distal section, and the pull wire is connected to the anchor ring and the operating handle respectively.

Optionally, the anchor ring is adjacent to the connection of the transition segment with the straight segment.

Optionally, the anchor ring also serves as an imaging mark.

Optionally, wherein a distal end of the straight segment is further provided with an imaging mark.

Optionally, the pull wire is deflected around a central axis of the flexible catheter.

Optionally, the pull wire is deflected by 90° around the central axis of the flexible catheter.

Optionally, a start point of the guiding passageway at the distal section is A, point B is deflected by 90° relative to the point A in a circumferential direction of the flexible catheter, and a transitional guiding section is formed between point A and point B, wherein the transitional guiding section extends along a part or an entire of the trans-aortic arch section, or a part or an entire the main section.

Optionally, the transitional guiding section has a length of L, where 0 mm < L ≤ 250 mm.

The present disclosure discloses a transcatheter treatment system, including the adaptive guiding device as mentioned above and a treatment device movably received in the flexible catheter, wherein the flexible catheter functions to establish a path from outside a human body to inside the human body, and the treatment device is configured to pass through an aortic arch via the flexible catheter.

Optionally, the treatment device is selected from at least one of a myocardial injection device, a myocardial ablation device, a valve repair device, or a valve replacement device.

Optionally, the treatment device includes a bending sheath, an inner sheath and a treatment assembly, wherein the bending sheath is movably inserted in the flexible catheter and is bendable in a single direction, the inner sheath is movably inserted in the bending sheath, and the treatment assembly is inserted in the inner sheath.

Optionally, a bending direction of the bending sheath is different from a bending direction of the distal section of the flexible catheter of the adaptive guiding device.

Optionally, the bending direction of the bending sheath is substantially perpendicular to the bending direction of the distal section of the flexible catheter of the adaptive guiding device.

Optionally, a distal end of the inner sheath is fixedly connected with a limiting element, and after the inner sheath extends from a distal end of the bending sheath, the limiting element is configured to contact a tissue surface.

Optionally, the treatment assembly is selected from at least one of an injection assembly, an ablation assembly, a prosthetic heart valve, an annuloplasty ring, a valve clamping device, a suture, and a tissue anchor or a tissue puncture member. The embodiments of the present disclosure at least have the following benefits: by providing the guiding device with a flexible catheter which is pre-shaped and bendable, the adaptability of the guiding device to the blood vessel morphology can be improved, in its natural state, it can better adapt to the complex anatomical morphology of the human aortic arch, thereby simplifying the guidance operation, reducing the pressure to human blood vessels, and improving the reliability of the device. In addition, the structure of the guiding device is simplified, the guiding device in use does not require a large avoidance space, so that the guiding device has a larger operation and adjustment space and a more convenient initial position for adjustment, and the tissue can be prevented from extending into the avoidance area, thereby reducing blood vessel damage and improving the operation safety of the device.

### BRIEF DESCRIPTION OF DRAWINGS

FIG. 1 is a schematic view of an overall adaptive guiding device according to an embodiment of the present disclosure;
FIG. 2 is a schematic partial view of a flexible catheter according to an embodiment of the present disclosure;
FIG. 3 is a schematic partial front view of the flexible catheter according to an embodiment of the present disclosure;
FIG. 4 is a schematic partial side view of the flexible catheter according to an embodiment of the present disclosure;
FIG. 5 is a schematic partial bottom view of the flexible catheter according to an embodiment of the present disclosure;
FIG. 6 is a schematic view showing the flexible catheter according to an embodiment of the present disclosure crossing the aortic arch of the heart;
FIG. 7 is a schematic view showing the flexible catheter according to an embodiment of the present disclosure in the heart;
FIG. 8 is a schematic view showing the assembly of the flexible catheter and the pull assembly according to an embodiment of the present disclosure;
FIG. 9 is a cross-sectional view of section C-C in FIG. 8;
FIG. 10 is an enlarged view of part M in FIG. 9;
FIG. 11 is a cross-sectional view of section D-D in FIG. 8;
FIG. 12 is a schematic view showing the bend direction of the flexible catheter according to an embodiment of the present disclosure;
FIG. 13 is a schematic view of the flexible catheter according to an embodiment of the present disclosure when used in the heart and being bent;
FIG. 14 is a schematic view of an overall transcatheter endocardium injection system according to an embodiment of the present disclosure;
FIG. 15 is an enlarged view of part N in FIG. 14;
FIG. 16 is an exploded view of a transcatheter endocardium injection system according to an embodiment of the present disclosure;
FIG. 17 is an exploded view of a transcatheter endocardium injection system according to another embodiment of the present disclosure;
FIG. 18 is a schematic view showing a needle of a transcatheter endocardium injection system according to an embodiment of the present disclosure penetrated into the free wall;
FIG. 19 is a schematic view showing the bend direction of a bending sheath and the flexible catheter of the adaptive guiding device of the transcatheter endocardium injection system according to an embodiment of the present disclosure;
FIG. 20 is a schematic view showing a first state in which the transcatheter endocardium injection system according to an embodiment of the present disclosure is delivered into the left atrium of the heart;
FIG. 21 is an enlarged view of part O in FIG. 19;
FIG. 22 is a schematic view showing a second state in which the transcatheter endocardium injection system according to an embodiment of the present disclosure is delivered into the left atrium of the heart;
FIG. 23 is a further exemplary structural view of a transcatheter endocardium injection system according to an embodiment of the present disclosure;
FIGS. 24 to 28 are schematic views showing the process of using the transcatheter treatment system according to an embodiment of the present disclosure;
FIG. 29 is a schematic view of a transcatheter treatment system according to an embodiment of the present disclosure using a myocardial ablation device as a treatment device; and
FIGS. 30 to 31 are schematic views showing imaging marks according to an embodiment of the present disclosure.

### List of reference signs:

1, adaptive guiding device; 101, flexible catheter; 1011, main section; 1012, trans-aortic arch section; 10121, first arc segment; 10222, second arc segment; 10123, third arc segment; 10124, fourth arc segment; 1013, distal section; 10131, transition segment; 10132, straight segment; 102, operating handle; 103, pull assembly; 1031, pull wire; 1032, guiding passageway; 10321, transitional guiding section; 1033, anchor ring; 2, treatment device; 201, bending sheath; 202, inner sheath; 2021, metal sleeve; 203, treatment assembly; 2031, injection channel; 2032, needle; 204, first adjustment handle; 205, second adjustment handle; 3, aortic arch; 4, left ventricle; 5, anterior papillary muscle; 6, posterior papillary muscle; 7, target area; 8, aortic valve; 9, free wall; 10, first plane; 11, second plane; 12, third plane; 13, ablation needle; 14, ablation energy generating device; 15, perfusion device.

### DESCRIPTION OF EMBODIMENTS

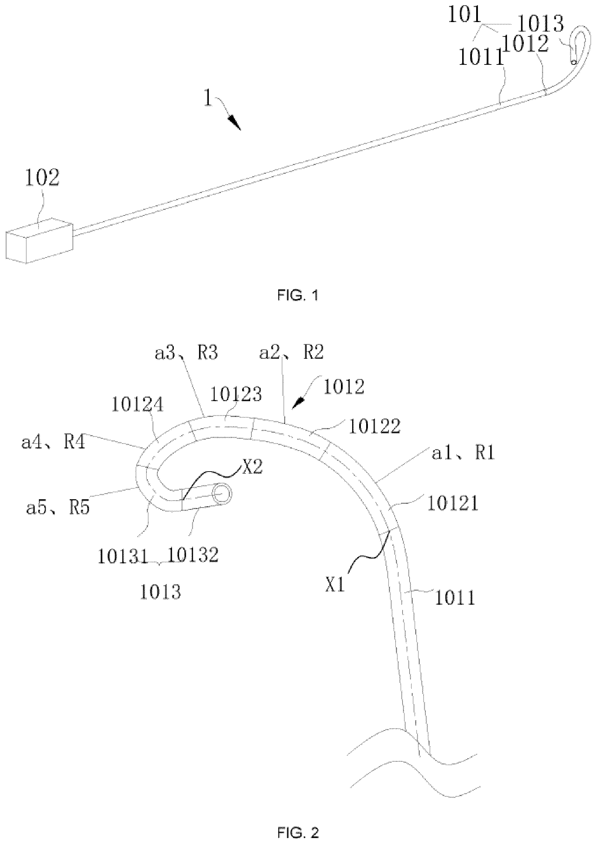

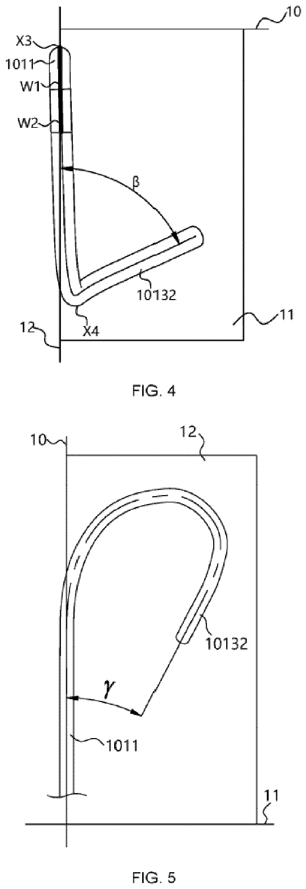

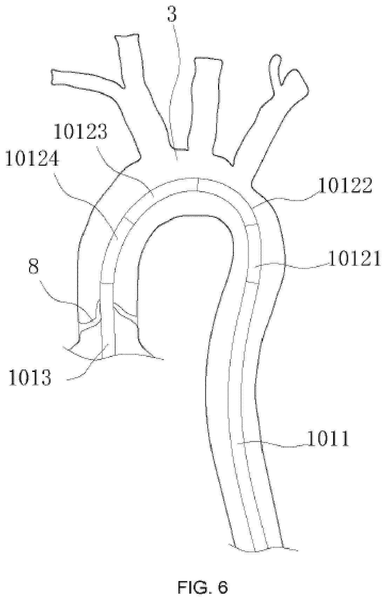

Referring to FIGS. 1 to 7, some embodiments disclose an adaptive guiding device 1, which includes a hollow flexible catheter 101 of a certain length. The flexible catheter 101 has been pre-shaped, and has bends in its natural state.

The distal part of the entire flexible catheter 101 is a distal section 1013. The distal section 1013 at least includes a transition segment 10131 and a straight segment 10132. The transition segment 10131 is bendable.

Regarding the bending method of the flexible catheter 101, for example, it is bent at least five times relative to its own central axis in different planes, wherein the angle between the two planes of one bend closest to the distal end is greater than 90°, and the angles between the planes of at least four consecutive bends is less than 90°, so that at least part of the flexible catheter can conform to the anatomical shape of the aortic arch.

The angle between the planes of at least four consecutive bends being less than 90° is explained referring to the arc segments below. For example, the first arc segment 10121, the second arc segment 10222, the third arc segment 10123, and the fourth arc segment 10124 each define a plane, and the angle (deflection amplitude) between the planes where adjacent arc segments are located is less than 90°. In the figure, the first arc segment 10121 is located on the plane W1, the second arc segment 1022 is located on the plane W2, and the angle between the plane W1 and plane W2 is less than 90°. The same applies to the other arc segments. Since the arc segments are not coplanar, bending in different planes is achieved.

The angle between the two planes of one bend closest to the distal end is greater than 90°.

By providing the guiding device with a flexible catheter 101 which is pre-shaped with a specific shape, the distal end of the flexible catheter 101 in a natural state can conform to the anatomical shape of the aortic arch of the human body, thereby simplifying the guiding operation, improving the reliability of the device, reducing the pressure to human blood vessels and thus reducing the damage to blood vessels, and improving the operation safety of the treatment device.

In this embodiment, in order to provide the adaptive guiding device 1 with strong compliance in human blood vessels, as well as certain pushability and twist resistance, the flexible catheter 101 preferably uses a pre-shaped multi-layer sheath structure. The adaptive guiding device 1 is an adaptive guiding sheath. Pre-shaping refers to the process of placing the flexible catheter in a mold with a specific shape and heating it to a certain temperature to form the flexible catheter into a specific shape. In this embodiment, pre-shaping specifically includes the following steps: placing the multi-layer sheath in the mold the inner cavity of which corresponding to the anatomical shape of the aortic arch, heating the placed multi-layer sheath and the mold together, cooling to room temperature, and de-molding. The heating temperature and time depend on the material and structure of the sheath.

In other embodiments, the flexible catheter 101 can be a metal cut tube, a metal wire braided tube, or a flexible tube made of other polymer materials, which only needs to be pre-shaped so that the catheter has a specific shape adapted to the anatomical shape of the aortic arch.

Further, in this embodiment, the multi-layer sheath structure of the flexible catheter 101 includes at least three layers, including an inner layer (not shown in the figure), a middle layer (not shown in the figure) and an outer layer (not shown in the figure) arranged sequentially from the inside to the outside. The inner layer is a polymer inner film, which can be made of PTFE (polytetrafluoroethylene). The middle layer is a metal braided mesh, which can be stainless steel mesh or tungsten wire mesh. The outer layer is a polymer outer film, and can be optionally made of PEBAX. Depending on the curvature of blood vessels, the hardness of the outer film of the multi-layer sheath at different parts can be varied to adapt to the curvature of different blood vessels, and to improve the pushability and twist resistance of the sheath. For example, the commonly used hardness specifications of PEBAX are 25D, 35D, 55D, 72D, etc. PEBAX with different hardness can be used for different parts of the outer film of the catheter as needed to meet the performance requirements of different parts of the catheter.

Referring to FIGS. 1 to 7, furthermore, the flexible catheter 101 includes a main section 1011, a trans-aortic arch section 1012 and a distal section 1013 connected in sequence from the proximal end to the distal end, wherein the trans-aortic arch section 1012 has a curved structure simulating the anatomical shape of the aortic arch 3. The main section 1011 is the starting section of the overall flexible catheter 101 and provides support for the overall structure. The trans-aortic arch section 1012 simulates the anatomical shape of the aortic arch 3 and is configured to traverse the aortic arch 3 of the human heart, to improve the compliance with the aortic arch 3 and reduce the pressure to vessels. The distal section 1013 is an output end of the catheter and configured to provide guidance for the treatment device installed in the flexible catheter 101.

For ease understanding and simple description, the side close to the main section 1011 is designated as the proximal end of the flexible catheter 101, and the side close to the distal section 1013 is designated as the distal end of the flexible catheter 101. The treatment device is inserted from the proximal end of the flexible catheter 101 and extends out from the distal end, that is, the lumen inlet of the flexible catheter 101 is located at its proximal end, and the lumen outlet is located at its distal end.

The main section 1011 is usually a straight section or an approximately straight section, and mainly functions to traverse the femoral artery and descending aorta to reach the start of the turning part of the aortic arch 3. Therefore, it is the longest section of the guiding sheath, and the length can be in the range of 540 mm to 840 mm.

In order to avoid twisting or bending of the flexible catheter 101 in curved blood vessels and to increase the twist resistance of the flexible catheter 101, the main section 1011 usually has a high hardness. In this embodiment, the main section 1011 preferably has a PEBAX outer film with a hardness of 72D. The trans-aortic arch section 1012 is connected to the main section 1011 and the distal section 1013 for traversing and conforming to the anatomical shape of the aortic arch 3.

In order to improve the compliance with the aortic arch 3 and reduce the pressure to the blood vessels, the trans-aortic arch section 1012 uses a curved arc segment simulating the shape and curvature of the aortic arch 3. Since the aortic arch 3 is a three-dimensional arch, the more the arc segments that simulate the aortic arch, the better the guiding device can conform to the aortic arch 3. However, the processing difficulty will be increased accordingly. Further, too many arc segments will cause the tube of the trans-aortic arch section 1012 to be stiffer, reducing crossability, and affecting the adaptability.

Referring to FIG. 2, further, in this embodiment, the trans-aortic arch section 1012 at least includes a first arc segment 10121, a second arc segment 10222, a third arc segment 10123 and a fourth arc segment 10124 that are sequentially connected and tangent one after another. The first arc segment 10121, second arc segment 10222, third arc segment 10123 and fourth arc segment 10124 are located on different planes. The first arc segment 10121 is connected with and communicated to the main section 1011, and the fourth arc segment 10124 is connected with and communicated to the distal section 1013.

Therefore, the fitness or conformity between the trans-aortic arch section 1012 of the flexible catheter 101 and the aortic arch 3 is higher, the adaptability and crossability are better, and the pressure to blood vessels can be reduced to reduce blood vessel damage.

Further, the angle of the first arc segment 10121 is a1, and the radius of the arc bend of the first arc segment 10121 is R1; the angle of the second arc segment 10222 is a2, and the radius of the arc bend of the second arc segment 10222 is R2; the angle of the third arc segment 10123 is a3, and the radius of the arc bend of the third arc segment 10123 is R3; the angle of the fourth arc segment 10124 is a4, and the radius of the arc bend of the fourth arc segment 10124 is R4.

4.5° ≤ a1 ≤ 30°, 90 mm ≤ R1 ≤ 450 mm; 25° ≤ a2 ≤ 80°, 10 mm ≤ R2 ≤60 mm; 20° ≤ a3 ≤ 70°, 25 mm ≤ R3 ≤ 50 mm; 20° ≤ a4 ≤ 110°, 20 mm ≤ R4 ≤ 50 mm.

The above parameters are obtained by simulation calculation based on the common structure of the aortic arch 3. With the above parameters, the trans-aortic arch section 1012 can better conform to the anatomical shape of the aortic arch 3 of most human hearts, and the pressure to blood vessels can be significantly reduced, in order to reduce blood vessel damage and improve the operation safety of the treatment device.

In addition, in order to provide the trans-aortic arch section 1012 after shaping with a certain hardness and prevent the tube of the trans-aortic arch section 1012 from bending when being pressed by the blood vessel wall, or prevent the trans-aortic arch section 1012 from being stretched when delivering the treatment device, in this embodiment, the hardness of the PEBAX outer film of the trans-aortic arch section 1012 is preferably 55D.

Referring to FIGS. 2 to 5, further, the distal section 1013 at least includes a transition segment 10131 and a straight segment 10132. The transition segment 10131 is arc-shaped, and its two ends are respectively tangent to the straight segment 10132 and the trans-aortic arch section 1012. This can avoid sharply changes in the curvature of the tube of the distal section 1013 and reduce the resistance and difficulty of the treatment device passing through the guiding device.

The at least five bends obtained by the pre-shaping mentioned above can be understood as four of them correspond to the first arc segment 10121, the second arc segment 10222, the third arc segment 10123 and the fourth arc segment 10124, and the fifth bend corresponds to the transition segment 10131 that is pre-shaped into an arc shape.

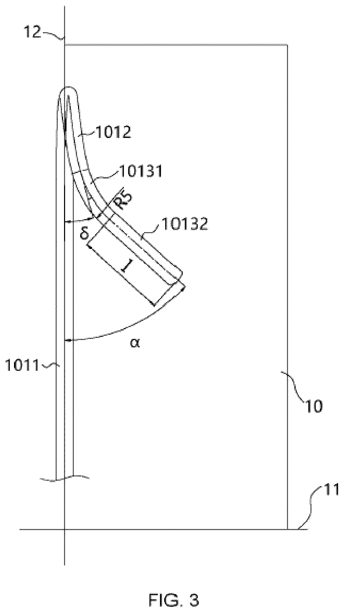

Further, as shown in FIG. 3, the angle of the transition segment 10131 is a5, and the radius of the arc bend of the transition segment 10131 is R5 ( projection on the first plane 10), where 15° ≤ a5 ≤ 70°, and 25 mm ≤ R5 ≤ 45 mm.

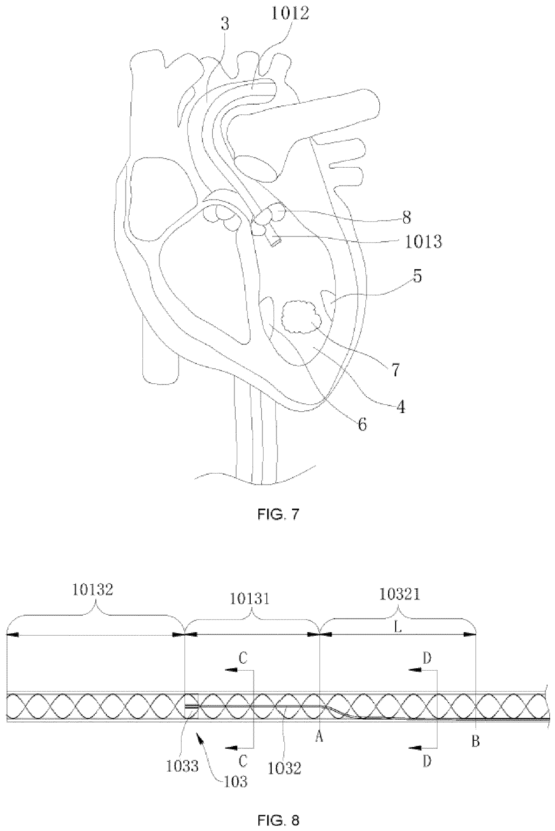

Referring to FIG. 7, the angle a5 of the transition segment 10131 and the radius R5 of the arc bend thereof affect the orientation of the straight segment 10132. Further, the bending radius of the transition segment 10131 will also affect the bending force. The larger the bending radius, the smaller the rigidity of the transition segment 10131, and the smaller the bending force required when bending. However, the bending radius should not be too large otherwise it would affect the crossability of the tube of the transition segment 10131. Therefore, in this embodiment, based on the above settings, the distal section 1013 in the initial state can point toward the middle position between the anterior papillary muscle 5 and the posterior papillary muscle 6, so that the treatment device that passes through the flexible catheter 101 and enters the heart can have a larger operation and adjustment space and a convenient initial position for adjustment, that is, the delivered treatment device has a wider operation range and coverage, while avoiding affecting the crossability of the tube of the transition segment 10131 and the bending force.

Referring to FIGS. 3 to 5, further, the angles between the central axis of the projection of the straight segment 10132 on the first plane 10, the second plane 11, and the third plane 12, respectively, and the central axis of the main section 1011 are α, β, γ. The first plane 10 is a vertical plane, the second plane 11 is a perpendicular plane perpendicular to the vertical plane, and the third plane 12 is a horizontal plane perpendicular to both the vertical plane and the perpendicular plane, where 35° ≤ α ≤ 85 °, 25° ≤ β ≤ 80°, and 20° ≤ γ ≤ 75°.

It can be seen from the figure that the first plane 10 and the third plane 12 intersect perpendicularly. For ease of understanding, the perpendicular intersection position is roughly the central axis of the main section 1011. The angles a1 to a4 of the first to fourth arc segments can be understood as the corresponding central angles of projections of the segments on the third plane 12.

As shown in the figure, taking the angle of the first arc segment 10121 as an example, by projecting the first arc segment 10121 on the third plane, the angle a1 can be determined according to its radius R1.

Referring to FIG. 7, the angles α, β, and γ jointly affect the orientation of the straight segment 10132 at the distal end of the flexible catheter 101. The angle α determines the distance between the distal end of the flexible catheter 101 and the anterior or posterior papillary muscles 5 or 6 in the heart. The smaller the α, the closer the straight segment 10132 of the flexible catheter 101 is to the posterior papillary muscle 6; and the greater the α, the closer the straight segment 10132 of the flexible catheter 101 is to the anterior papillary muscle 5. The angles β and γ determine the distance between the straight segment 10132 of the flexible catheter 101 and the interventricular septum or free wall 9 in the heart. The smaller the β and γ, the closer the straight segment 10132 of the flexible catheter 101 is to the free wall 9; and the greater the β and γ, the closer the straight segment 10132 of the flexible catheter 101 is to the interventricular septum. In this embodiment, by setting α, β and γ in the above ranges which affect the orientation of the end of the straight segment 10132 of the flexible catheter 101, the end of the straight segment 10132 of the flexible catheter 101 can accurately point toward the middle position of the area in front of the papillary muscles, preventing the end of the straight segment 10132 after entering the heart from being initially positioned in other areas except the middle position between the anterior papillary muscle 5 and the posterior papillary muscle 6, so that the straight segment 10132 of the flexible catheter 101 can be located in an appropriate position after entering the heart, and thus the straight segment 10132 of the flexible catheter 101 is easier to point to the target area 7 and quickly lock it.

Referring to FIG. 3 and FIG. 7, further, the length of the straight segment 10132 is I, where 0 < I < 80mm. In this embodiment, the length of the straight segment 10132 is preferably 40 mm. Within the suitable range, the longer the length of the straight segment 10132, the more accurate the orienting of the treatment device extending out of the straight segment 10132. In this embodiment, the above range allows the treatment device passing through the straight segment 10132 to accurately reach the target area 7.

In order to provide the trans-aortic arch section 1012 after shaping with a certain hardness and prevent the tube of the trans-aortic arch section 1012 from bending when being pressed by the blood vessel wall, or prevent the trans-aortic arch section 1012 from being stretched when delivering the treatment device, in this embodiment, the hardness of the PEBAX outer film of the transition segment 10131 and the straight segment 10132 of the distal section 1013 is preferably 25D.

Further, the angle between the central axis of the projection of the transition segment 10131 on the first plane 10 and the central axis of the main section 1011 is δ, where the first plane 10 is a vertical plane, and 0 < δ ≤ 45°.

The angle δ affects the initial position of the transition segment 10131 of the distal section 1013. Based on the above settings, it can be ensured that when the adaptive guiding device 1 enters the left ventricle 4, the trans-aortic arch section 1012 of the shaped flexible catheter 101 will be located at the middle position of the aortic arch 3, which provides enough space for the flexible catheter 101 to be displaced laterally during the adjustment process, and also greatly reduces the pressure to the blood vessels and aortic valve 8 in the heart.

Referring to FIG. 1, the adaptive guiding device 1 further includes an operating handle 102, which is connected to the main section 1011 and used to drive the flexible catheter 101 to move axially and circumferentially.

Specifically, the operating handle 102 is convenient for the operator to hold and operate, so that the flexible catheter 101 can move axially and circumferentially, and thus the distal section 1013 of the flexible catheter 101 can accurately point to the target area 7, providing accurate guidance for the treatment device. The operating handle 102 can be a conventional handle.

In the technical solution according to this embodiment, the flexible catheter 101 is pre-shaped so that the flexible catheter 101 in a natural state is bent at least five times relative to the central axis in different planes, thereby improving the adaptability of the guiding device to the vascular morphology. The guiding device can better adapt to the complex anatomical morphology of the human aortic arch 3, thereby simplifying the guidance operation, reducing the pressure to human blood vessels, and improving the reliability of the device. In addition, the structure of the guiding device is simplified, and the operation safety of the treatment device is improved. Further, the guiding device in use does not require a large avoidance space, so that the guiding device has a larger operation and adjustment space and a more convenient initial position for adjustment, and the tissue can be prevented from extending into the avoidance area, thereby reducing blood vessel damage and improving the operation safety of the treatment device.

Referring to FIG. 8, in some embodiments, the adaptive guiding device 1 further includes a pull assembly 103 for accurately adjusting the lateral displacement of the distal section 1013 of the flexible catheter 101 to further improve the adaptability to the vascular morphology.

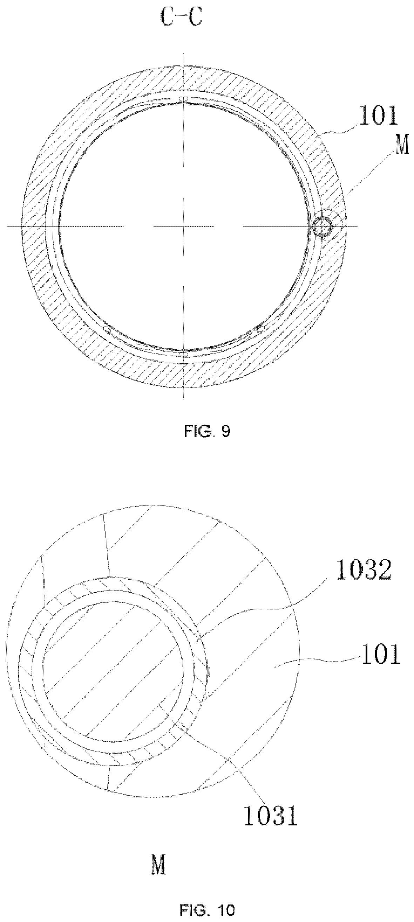

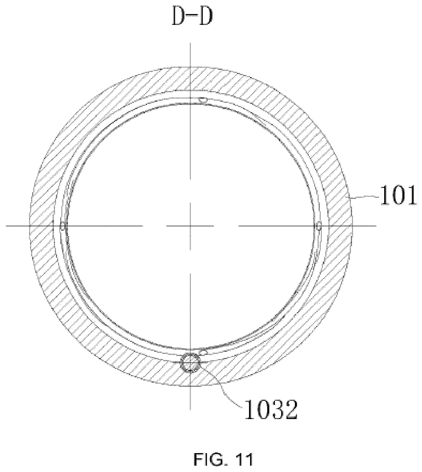

Referring to FIGS. 8 to 11, the pull assembly 103 includes a pull wire 1031, a guiding passageway 1032 and an anchor ring 1033. The guiding passageway 1032 is defined in the wall of the flexible catheter 101, that is, the guiding passageway 1032 is defined between the outer layer and the inner layer of the flexible catheter 101, specifically between the inner layer and the middle layer, or between the middle layer and the outer layer. The guiding passageway 1032 is configured for the pull wire 1031 to pass through. The anchor ring 1033 is fixedly connected to the transition segment 10131 of the distal section 1013, and the pull wire 1031 is fixedly connected to the anchor ring 1033 and the operating handle 102 respectively.

The pull wire 1031 is a flexible filament with a certain length and toughness, and can be selected from stainless steel braided ropes, non-metallic braided ropes (such as commonly used sutures), steel wires, nickel-titanium wires, etc. In this embodiment, stainless steel braided wires are preferred due to good toughness, bending resistance and flexibility.

The guiding passageway 1032 is defined in the wall of the flexible catheter and is used for the pull wire 1031 to pass through and protect the pull wire 1031. The guiding passageway 1032 can be a PI (polyimide) pipe, a PEEK (poly(ether-ether-ketone)) pipe, a flexible stainless steel cut tube, etc.. However, if the material used for the passageway of the pull wire 1031 is too hard, it will increase the resistance to the flexible catheter 101 during bending and lateral displacement. In this embodiment, the guiding passageway preferably uses a PI pipe.

Specifically, the operating handle 102 pulls the anchor ring 1033 through the pull wire 1031 of the pull assembly 103, thereby pulling the distal section 1013 of the flexible catheter 101, so as to control the movement amplitude and direction of the lateral displacement of the distal section 1013 of the flexible catheter 101, to ensure that the distal section 1013 of the flexible catheter 101 is located at the outflow tract of the aortic arch 3, providing a large space for bending and facilitating further clinical operations.

Referring to FIG. 8, further, the anchor ring 1033 is fixed at the transition segment 10131 of the distal section 1013.

That is to say, the transition segment 10131 serves as the to-be-bent segment. The pull wire 1031 is pulled by the operating handle 102, so that the transition segment 10131 can be bent accordingly, thereby driving the straight segment 10132 to displace laterally, so as to control the movement amplitude and direction of the lateral displacement of the distal section 1013 of the flexible catheter 101. Further, by designing the transition segment 10131 as the to-be-bent segment, as the end thereof is connected with the straight segment 10132, only a slight bending to the transition segment 10131 would cause a significant positional change of the end of the straight segment 10132. That is, only a small bending force is required in order to cause a significant positional change of the end of the distal section 1013 of the flexible catheter 101. Therefore, the movement space required by the flexible catheter 101 itself is reduced, and the output end of the distal section 1013 of the flexible catheter 101 can be adjusted more accurately and reliably.

Referring to FIG. 8, further, the anchor ring 1033 is fixedly connected to one end of the transition segment 10131 close to the straight segment 10132.

By designing the main section 1011 as the basis for rotation, the pull torque can be maximized and the pull force required for adjustment of the straight segment 10132 can be reduced.

Furthermore, the anchor ring 1033 can be made of stainless steel. In the case where the anchor ring 1033 is made of stainless steel, it can be fixedly connected to the pull wire 1031 by welding. In some embodiments, a single pull wire 1031 can be arranged in the guiding passageway 1032. By welding, one end of the single pull wire 1031 can be fixed to the anchor ring 1033, and the other end can be fixed to the operating handle 102.

The anchor ring 1033 can be made of other materials, in which case, the pull wire 1031 can be looped and connected to the anchor ring 1033.

In some embodiments, at least two pull wires 1031 are provided, that is, at least a first pull wire and a second pull wire are provided. The first pull wire and the second pull wire form a closed loop of a pull wire group, and one end of the pull wire group is connected to the anchor ring 1033, and the other end is connected to the operating handle 102.

Accordingly, the pull wire group can be pulled by the operating handle 102, so as to pull the distal section 1013 of the flexible catheter 101 to move in different directions.

Further, each pull wire 1031 is provided with a guiding passageway 1032, avoiding multiple pull wires 1031 being arranged in the same guiding passageway 1032 which would cause blockage or interference with each other, maintaining the reliability of adjustment, reducing the diameter of a single guiding passageway 1032 and reducing the profile of the entire flexible catheter 101.

In this embodiment, the pull wire 1031 and the anchor ring 1033 are preferably connected by welding, which can reduce the outer diameter of the whole of the pull wire 1031 and the anchor ring 1033, thereby reducing the resistance during lateral bending, and also reducing the outer diameter of the flexible catheter 101. The material of the anchor ring 1033 is preferably tantalum. Compared with stainless steel, tantalum has a better contrast effect under ultrasound.

Referring to FIG. 8, further, before the flexible catheter 101 is pre-shaped, in the direction from the distal section 1013 toward the main section 1011, the projection of the guiding passageway 1032 perpendicular to the central axis of the flexible catheter 101 gradually deflects around the central axis.

Therefore, compared with the case where the guiding passageway 1032 extends in a straight line parallel to the central axis of the flexible catheter 101 before the flexible catheter 101 is pre-shaped, in this embodiment, after the flexible catheter 101 is pre-shaped, the part of the guiding passageway 1032 from the distal section 1013 to the main section 1011 can deflect around the central axis, and the pull wire 1031 passing through the guiding passageway 1032 will deflect accordingly. Therefore, when the pull wire 1031 is pulled by the operating handle 102, the distal section 1013 can deflect better relative to the main section 1011 with the main section 1011 as the basis for deflection, thereby reducing the deflection of the main section 1011 in the same direction when adjusting the distal section 1013, and reducing the pressure to blood vessels.

Referring to FIGS. 8 to 11, further, the deflection start point of the guiding passageway 1032 at the distal section 1013 is A, and point B is deflected by 90° relative to the point A in the circumferential direction of the flexible catheter 101. A transitional guiding section 10321 is defined between point A and point B. The transitional guiding section 10321 extends along a part or the entire of the trans-aortic arch section 1012, or a part or the entire of the main section 1011.

That is, the terminal point of the deflected part of the guiding passageway 1032 can be set at the trans-aortic arch section 1012 or the main section 1011 in practice according to the desired operation effects.

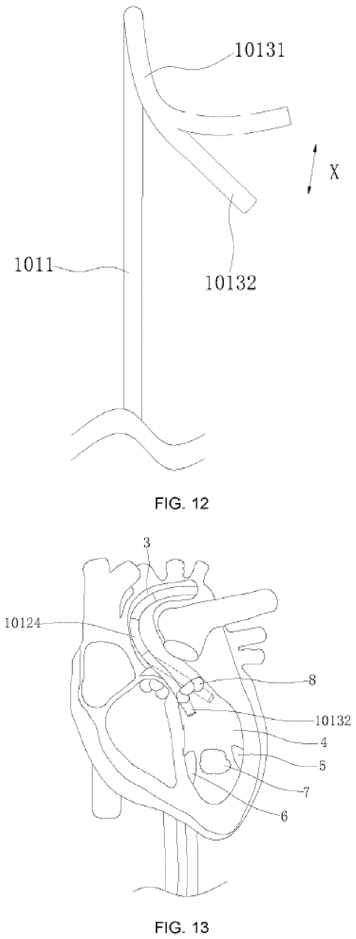

Referring to FIGS. 8 and 12, the length L of the transitional guiding section 10321 affects the deflection of the flexible catheter 101 during lateral bending. If the length of the transitional guiding section 10321 is too short, during lateral bending, a great bending force is required. If the length L of the transitional guiding section 10321 is too long, for example, if the entire pull wire 1031 extends and deflects from the point A to the operating handle 102, the entire flexible catheter 101 would be prone to deflect toward the pull wire 1031 when adjusting the distal section 1013 of the flexible catheter 101, increasing the deflection amplitude of the flexible catheter 101, increasing the pressure to the blood vessels, and easily causing damage to the blood vessels. In the present disclosure, the length L of the transitional guiding section 10321 is in the range of 0 mm < L ≤ 250 mm.

Furthermore, due to the high hardness of the PEBAX outer film of the main section 1011, the pull wire 1031 has a small impact on the main section 1011. Therefore, the length of the transitional guiding section 10321 mainly affects the pull force for the trans-aortic arch section 1012 and the deflection amplitude of the trans-aortic arch section 1012 during bending. In this embodiment, the length of the transitional guiding section 10321 is preferably 40 mm, so that the pull force for the trans-aortic arch section 1012 and the deflection amplitude of the trans-aortic arch section 1012 during bending can be appropriate.

Since the transition segment 10131 of the distal section 1013 serves as the to-be-bent segment for lateral displacement, the hardness of the PEBAX outer film is proportional to the bending force of the pull wire 1031. The lower the hardness of the PEBAX outer film, the smaller the bending force of the pull wire 1031 when the distal end of the flexible catheter is displaced.

Therefore, the PEBAX outer film of the transition segment 10131 is preferably 25D, which not only requires a small bending force, but also ensures a certain strength to prevent the tube of the transition segment 10131 from being pressed by the blood vessel wall and bent.

In addition, since the flexible catheter 101 in this embodiment needs to displace laterally in use, it is necessary to provide a fulcrum for the flexible catheter 101 at the aortic arch 3 so that the part of the flexible catheter 101 in front of the fulcrum remains motionless, while in the other part in rear of the fulcrum, the distal section 1013 of the flexible catheter 101 can be displaced laterally by bending, thereby displacing a specified section in a specified direction.

Specifically, as shown in FIGS. 12 and 13, the fourth arc segment 10124 of the trans-aortic arch section 1012 serves as a fulcrum to abut against the outside of the aortic arch 3, so that when the flexible catheter 101 is displaced laterally, the flexible catheter 101 has an appropriate support force, while the first arc segment 10121, the second arc segment 10222, and the third arc segment 10123 are not in contact with the inside of the aortic arch 3, thereby reducing the pressure to blood vessels.

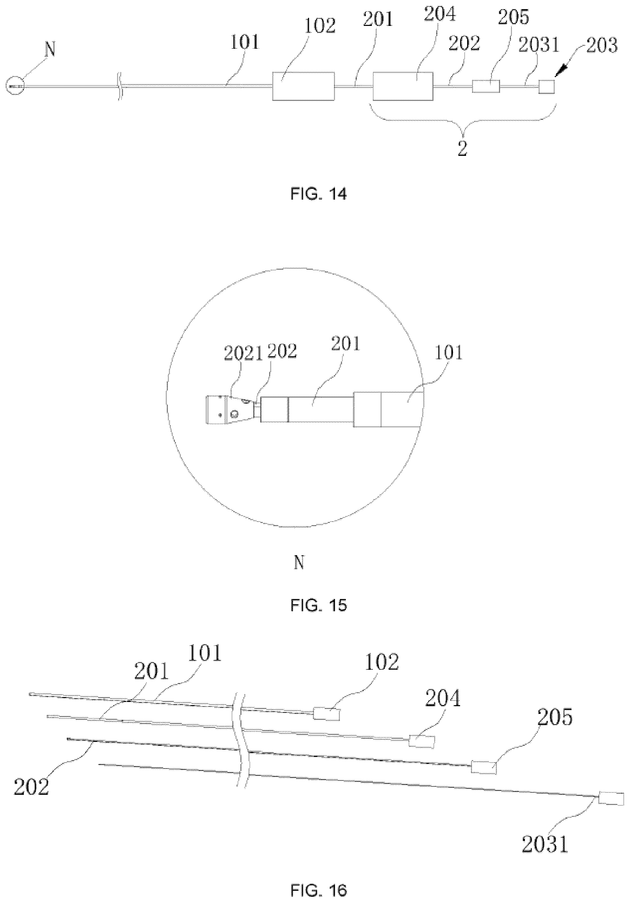

Referring to FIG. 14, in some embodiments, a transcatheter treatment system is disclosed, which includes the adaptive guiding device 1 according to any of the above embodiments, and further includes a treatment device 2 movably installed in the flexible catheter 101. The adaptive guiding device 1 functions to establish a path for intervention from the exterior of the human body into the interior of the human body through the aorta, and the treatment device 2 passes through the aortic arch through the flexible catheter 101. The flexible catheter 101 of the adaptive guiding device 1 serves as the outermost sheath (outer sheath in short) of the entire transcatheter treatment system, and usually cooperates with the guide wire to establish the path from the exterior of the human body into the interior of the human body.

By applying the improved adaptive guiding device 1 into the transcatheter treatment system, the guidance efficiency can be improved and the operation safety risks can be reduced.

Further, the treatment device is selected from at least one of a myocardial injection device, a myocardial ablation device, a valve repair device or a valve replacement device. That is, the adaptive guiding device 1 can be applied in transcatheter endocardium injection systems, transcatheter radiofrequency ablation systems, or heart valve repair systems, to improve guidance efficiency and reduce operation safety risks of the treatment device.

Referring to FIGS. 14 to 16, further, the treatment device 2 includes a bending sheath 201, an inner sheath 202 and a treatment assembly. The bending sheath 201 is movably inserted into the flexible catheter 101 and can be bent in a single direction. The inner sheath 202 is movably inserted into the bending sheath 201. The treatment assembly is inserted into the inner sheath 202. The treatment assembly is selected from at least one of an injection assembly, an ablation assembly, a prosthetic heart valve, an annuloplasty ring, a valve clamping device, a suture, a tissue anchor, or a tissue puncture member. For example, the treatment assembly is an injection assembly 203.

The bending sheath 201 is movably installed in the interior lumen of the flexible catheter 101 of the adaptive guiding device 1 and can be bent unidirectionally. The bending sheath 201 cooperates with the adaptive guiding device 1 to deliver the treatment assembly to the predetermined treatment site. The inner sheath 202 is movably installed in the interior lumen of the bending sheath 201 to prevent the treatment assembly from scratching the inner wall of the bending sheath 201 and to protect and ensure the movement of the treatment assembly.

Referring to FIG. 14, FIG. 15, FIG. 18 and FIG. 21, optionally, the distal end of the inner sheath 202 is fixedly connected to a limiting element. The limiting element can be a metal sleeve 2021. The end face of the metal sleeve 2021 is a plane with a certain area, in order to avoid damaging or puncturing the free wall 9 of the left ventricle 4 when it is in contact with the free wall 9.

The following describes a transcatheter treatment system of the present disclosure, taking the injection assembly 203 as the treatment assembly for example.

Referring to FIGS. 14, 16 to 18, and 20 to 23, a transcatheter endocardium injection system is shown for injecting hydrogel, cells and other therapeutic agents to diseased ischemic myocardial tissue through a catheter to improve heart function, and can be applied in heart failure treatment. The transcatheter endocardium injection system includes an adaptive guiding device 1 and a treatment device 2. The adaptive guiding device 1 serves as an outer sheath, and the treatment device 2 is inserted into the adaptive guiding device 1. The treatment device 2 includes an injection assembly 203. The injection assembly 203 includes an injection channel 2031 and a needle 2032 connected to and communicated with the distal end of the injection channel 2031. The needle 2032 is used to penetrate into the myocardial free wall 9 and inject therapeutic agents such as contrast medium, drugs, or hydrogels. The end plane of the metal sleeve 2021 at the distal end of the injection channel 2031 defines a through hole for the needle 2032 to pass through. The injection channel 2031 includes an inner channel (not shown in the figure) for drug injection and an outer channel for contrast medium injection. The contact of the metal sleeve 2021 with the free wall 9 can be judged by the diffusion of the contrast medium on the free wall 9.

Specifically, the treatment device 2 includes a bending sheath 201 inserted in the flexible catheter 101, an inner sheath 202 inserted in the bending sheath 201, and an injection assembly 203 inserted in the inner sheath 202. Under the main guidance of the outer sheath, with the bending sheath 201 as the intermediate support and auxiliary adjustment basis, the injection assembly 203 can pass through the inner sheath 202 and then inject therapeutic drugs or other agents into the target area 7 (myocardial tissue).

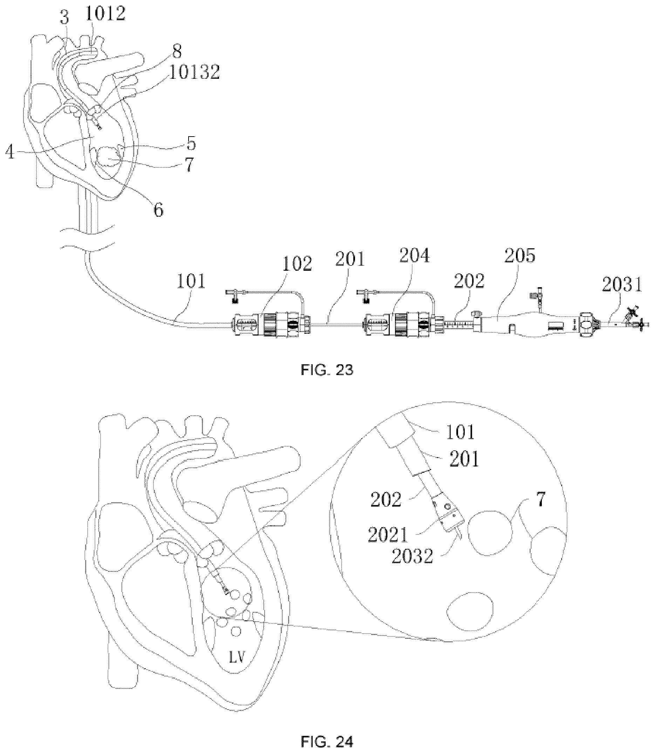

FIG. 23 is a further exemplary structural schematic view of the transcatheter endocardium injection system.

Referring to FIGS. 14, 16, 20-23, in order to facilitate the adjustment of the bending sheath 201 and the inner sheath 202, a first adjustment handle 204 and a second adjustment handle 205 are provided corresponding to the bending sheath 201 and the inner sheath 202 respectively. Both the first adjustment handle 204 and the second adjustment handle 205 can be conventional handles.

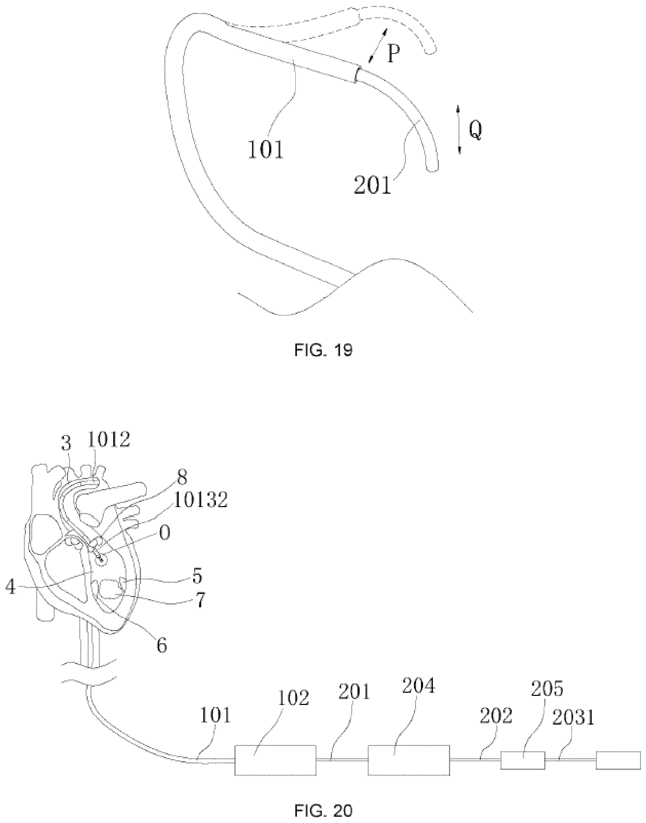

Referring to FIG. 19, further, the bend direction of the bending sheath 201 is different from the bend direction of the distal section 1013 of the flexible catheter 101 of the adaptive guiding device 1. By setting the bend direction of the bending sheath 201 to be different from the bend direction of the distal section 1013, the adjustment range of the orientation of the inner sheath 202 passing through the bending sheath 201 and thus the orientation of the treatment assembly passing through the inner sheath 202 can be enlarged. Therefore, the adjustment flexibility of the output end of the transcatheter treatment system can be improved, which is conducive to guiding the treatment assembly passing through the inner sheath 202 to the target area 7 more accurately, achieving precise guidance.

Referring to FIG. 19, further, the bend direction of the bending sheath 201 is substantially perpendicular to the bend direction of the distal section 1013 of the flexible catheter 101 of the adaptive guiding device 1.

As a preferred embodiment, the bending sheath 201 is arranged in the direction perpendicular to the bend direction of the distal section 1013, in order to improve the adjustment flexibility of the output end of the transcatheter treatment system and guide the treatment assembly passing through the inner sheath 202 to the target area 7 more accurately, achieving precise guidance.

For example, in use of the endocardium injection system of the present disclosure, the distal section 1013 of the outer sheath (i.e., the flexible catheter 101 of the adaptive guiding device 1) is displaced laterally, and the bend direction of the middle sheath (i.e., the bending sheath 201) that is inserted into the lumen of the outer sheath is the vertical direction perpendicular to the lateral displacement. The different bend directions of the two sheaths cooperating with each other further ensure that the injection assembly is delivered to the desired site for drug injection, so as to achieve good therapeutic effects.

In the following, the delivery path through the femoral artery - aortic arch 3 - left ventricle 4 is taken as an example to illustrate the use method of the transcatheter endocardium injection system according to this embodiment.

In step S1, as shown in FIG. 20, after puncture of the femoral artery, the adaptive guiding device 1 cooperates with the conventional guide wire to reach the left ventricle 4 through the aortic arch 3 via the femoral artery. Now, the flexible catheter 101 of the adaptive guiding device 1 is in the initial state, wherein the distal end of the tube of the adaptive guiding device 1 is centered at a certain distance below the aortic valve 8. Then the treatment device 2 is delivered into the left ventricle 4 along the flexible catheter 101 of the adaptive guiding device 1 until the metal sleeve 2021 at the distal end of the inner sheath 202 is exposed.

After the flexible catheter 101 reaches the predetermined position first, and then the treatment device 2 is delivered, which is conducive to taking advantage of the pre-shaping of the flexible catheter 101 and avoiding the impact on the pre-shaping if the flexible catheter 101 is delivered synchronously with the treatment device 2. Alternatively, the distal end of the flexible catheter 101 does not cross the valve, that is, it stays above the aortic valve 8 and does not extend into the left ventricle.

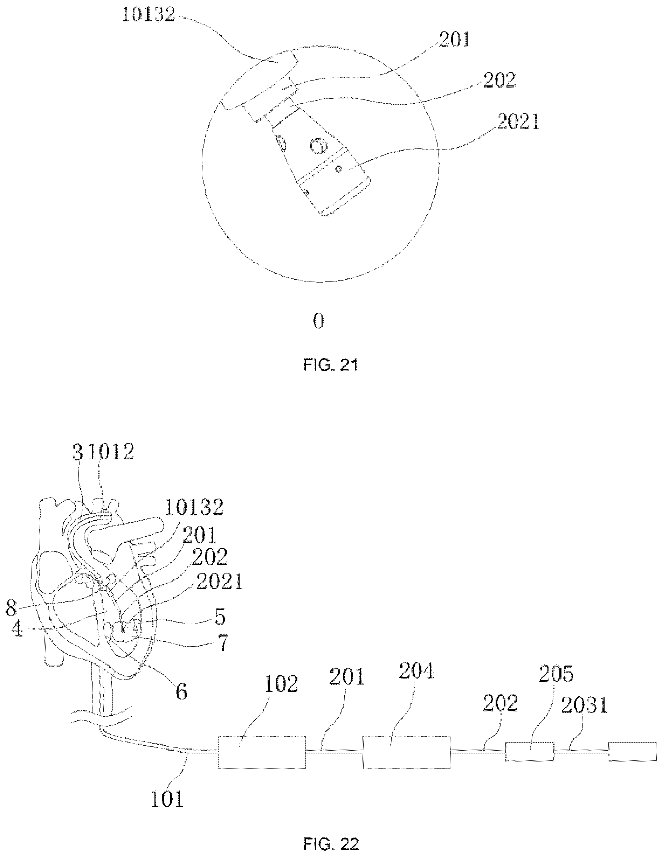

In step S2, as shown in FIGS. 20 and 22, the distal output end of the flexible catheter 101 is adjusted to point between the anterior and posterior papillary muscles 6. Now, the bending sheath 201 of the treatment device 2 is pushed forward so that the distal end of the bending sheath 201 is at a certain distance from the distal output end of the flexible catheter 101, and then the distal end of the bending sheath 201 is bent so that the metal sleeve 2021 at the distal end of the inner sheath 202 faces the target area 7 as perpendicularly as possible. Then, the inner sheath 202 is pushed further forward until the metal sleeve 2021 contacts the free wall 9 of the target area 7.

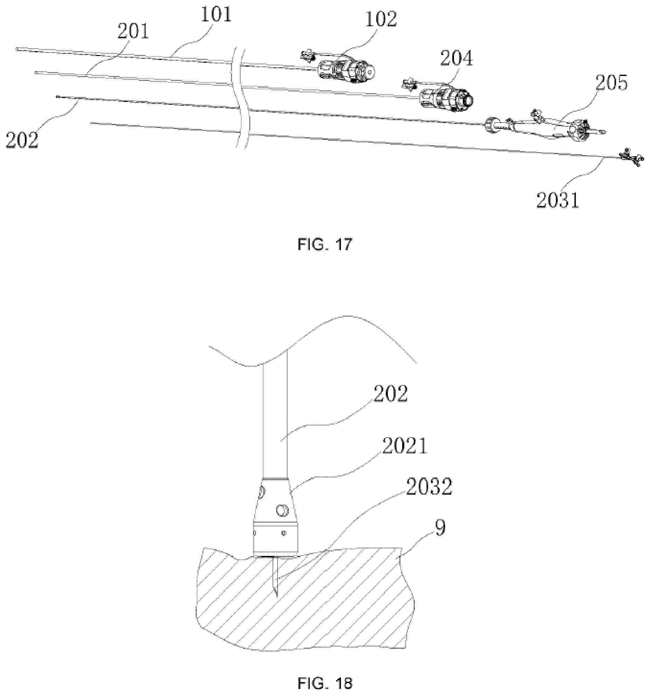

In step S3, as shown in FIG. 18, the needle 2032 at the distal end of the injection channel 2031 penetrates into the free wall 9. Now, the contrast medium can be injected into the outer channel of the injection channel 2031 and the diffusion of the contrast medium is observed. For example, if the contrast medium diffuses out from the circumferentially distributed through holes of the metal sleeve 2021, it can be determined that the needle 2032 has firmly penetrated into the treatment site. Now, the therapeutic agent such as hydrogel can be injected into the inner channel to fill the free wall 9 with the drug so as to achieve drug injection.

In step S4, if injection at multiple treatment sites is required, the needle 2032 is withdrawn so that the needle 2032 is retracted into the metal sleeve 2021; and the inner sheath 202 is withdrawn so that the metal sleeve 2021 contacts the distal end of the bending sheath 201. The bent configuration of the flexible catheter 101 is kept unchanged, the bending sheath 201 is rotated, and steps S1 to S3 are repeated so as to achieve hydrogel injection at other sites on the same level as the first site. Optionally, the extension of the flexible catheter 101 is increased or decreased, and the bending sheath 201 is rotated so as to select a higher or lower site with respect to the papillary muscle than the first site, and then steps S1 to S3 are repeated to achieve hydrogel injection.

In step S5, after completing the drug injection at all treatment sites, the needle 2032 is withdrawn, the injection channel 2031 is retracted, the bending sheath 201 is released, and the flexible catheter 101 is released. Finally, the treatment device 2 and the flexible catheter 101 are withdrawn.

For multi-point injection, in one embodiment, a method for controlling a transcatheter endocardium injection system is provided. The transcatheter endocardium injection system includes a flexible catheter 101, a bending sheath 201 and a needle 2032 slidably arranged sequentially from the outside to the inside, wherein the proximal end of the flexible catheter 101 is controlled by the operating handle 102, the proximal end of the bending sheath 201 is controlled by the first adjustment handle 204, and the proximal end of the needle 2032 is controlled by the second adjustment handle 205. The flexible catheter 101 and the bending sheath 201, driven by the corresponding handles, can be rotated and bent respectively.

The second adjustment handle 205 can be optionally connected to the inner sheath 202, which is located outside the needle 2032 and provided with a metal sleeve 2021 connected to the distal end of the inner sheath 202. In this case, the transcatheter endocardium injection system can use the relevant components in the above embodiments.

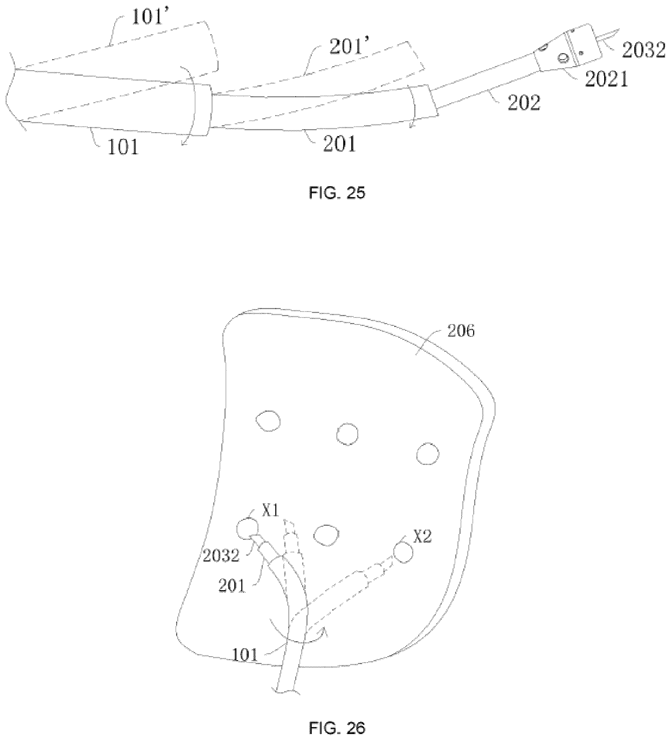

Referring to FIG. 24 and FIG. 25, the target area 7 is in the left ventricle LV. The flexible catheter 101 itself can be bent (for example, the flexible catheter 101 can be bent into the flexible catheter 101' as shown in FIG. 25), and can be driven by the operating handle 102 to rotate around its own longitudinal axis. Similarly, the bending sheath 201 itself can be bent (for example, the bending sheath 201 can be bent into the bending sheath 201' as shown in FIG. 25), and can be driven by the first adjustment handle 204 to rotate around its own longitudinal axis. In order to simplify the adjustment, the flexible catheter 101 after being adjusted in place can be kept stationary, and only the orientation and the distal/proximal position of the bending sheath 201 need to be adjusted. This allows for quick adjustment of the orientation and the position of the injection needle in specific cases of multi-point injection, thereby improving the surgical efficiency.

Referring to FIGS. 26 to 28, in the following embodiment, an operation method of the transcatheter treatment system is explained taking injection locations in a multiplerow-and-multiple-column arrangement as an example, that is, a position adjustment method of the needle for continuous injections is explained. The arrangement of injection locations in the figures is only an illustration, and can be adjusted in practice according to the area and location of the lesion, wherein the injection locations are the target area 7 and located on the left ventricular wall 206. In FIGS. 26 to 29, the inner sheath 202 and the metal sleeve 2021 are not shown, and during changing the injection location, the process of withdrawing the needle 2032 before changing the injection location and the process of advancing the needle 2032 after changing the injection location are not shown.

In injection, a method for controlling the transcatheter endocardium injection system according to this embodiment includes:
performing injection on a current injection location;
obtaining a spatial offset between the next injection location and the current injection location;
adjusting the needle position using at least one of the following methods according to the spatial offset until the needle corresponds to the next injection location; and
performing injection.

The spatial offset is realized by using at least one of the following methods so as to adjust the needle position until it corresponds to the next injection location. For example, the the needle position is adjusted by adjusting the flexible catheter 10 and/or adjusting the bending sheath 201, and specifically, by rotating, bending, moving distally or proximally as a whole. The various methods can be combined, and the adjustment magnitude can be controlled based on the modeling in combination with the real-time imaging device. The change method of injection location in different situations is explained by referring to the following exemplary operations. The same applies to other situations.

Referring to FIG. 26, the flexible catheter remains unchanged and the needle 2032 can be moved from the injection location X1 to the injection location X2 by simply rotating the bending sheath 201 around its longitudinal axis.

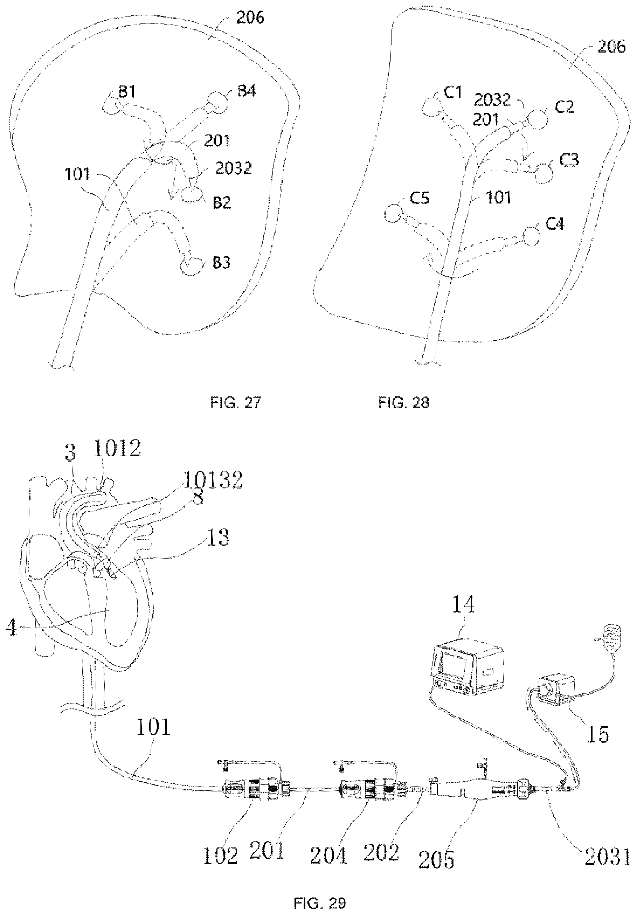

Referring to FIG. 27, the flexible catheter 101 remains unchanged, and the bending sheath 201 is rotated around its longitudinal axis, so that the needle 2032 moves from the injection location B1 to the injection location B2. The bending sheath 201 is bent to move the needle 2032 from the injection location B2 to the injection location B4.

Referring to FIG. 28, the flexible catheter 101 remains unchanged and the bending sheath 201 is rotated around its longitudinal axis to move the needle 2032 from the injection location C1 to the injection location C2 or C3. The bending sheath 201 remains bent and is retreated along the interventional path, so that the needle 2032 moves from the injection location C3 to the injection location C4. The bending sheath 201 is rotated around its longitudinal axis, so that the needle 2032 moves from the injection location C5 to the injection location C6.

In case of multiple injection locations, the bending sheath 201 is preferably rotated and bent to reduce axial movement along the interventional path, so as to reduce corresponding potential surgical risks.

It can be understood that the adaptive guiding device 1 of the present disclosure can be applied in different fields, such as transcatheter radiofrequency ablation systems, heart valve repair systems, etc., to establish lumens from the exterior of the human body to the interior of the human body for interventional devices as needed. The exemplary embodiments are not intended to limit the scope of the present disclosure.

For example, referring to FIG. 29, in the case where the adaptive guiding device 1 is applied to, for example, a transcatheter radiofrequency ablation system, an ablation assembly is used as a treatment assembly. The ablation assembly includes an ablation needle 13, an ablation energy generating device 14 connected to and providing ablation energy for the ablation needle 13, and a perfusion device 15 for providing electrolyte solution. Other structures of the transcatheter radiofrequency ablation system can refer to the above embodiments where the treatment assembly is an injection assembly, that is, the ablation assembly includes a bending sheath, an inner sheath and an ablation needle. The ablation assembly is movably inserted in the adaptive guiding device 1. After the ablation needle passes through the inner sheath, it enters the myocardial tissue by puncturing the endocardium, and then the energy generating device provides energy for the ablation needle to perform ablation on the myocardial tissue of the interventricular septum, thereby treating hypertrophic cardiomyopathy (HCM) through minimally invasive intervention.

It can also be understood that in the case where the adaptive guiding device 1 is applied in, for example, a transcatheter valve repair system, and an anchor is used as the treatment assembly, the anchor can be implanted in the ventricular wall through a catheter, and the anchor is connected with e-PTFE sutures, wherein one end of the suture is connected to the valve leaflet. The suture is used as an artificial chordae to realize the chordae tendineae repair of the valve. Alternatively, multiple anchors connected to each other through sutures can be implanted on the ventricular wall in sequence, and then the sutures are tightened to achieve ventricular volume reduction or valvuloplasty, thereby treating valvular regurgitation disease through minimally invasive intervention.

In summary, the adaptive guiding device 1 and the transcatheter treatment system including the adaptive guiding device 1 provided by the present disclosure can better adapt to the complex anatomical morphology of the aortic arch 3, and the structure of the guiding device is simplified, improving the operation safety of the treatment device. Further, the guiding device in use does not require a large avoidance space, so that the guiding device has a larger operation and adjustment space and a more convenient initial position for adjustment, and the tissue can be prevented from extending into the avoidance area, thereby reducing blood vessel damage and improving the operation safety of the treatment device.

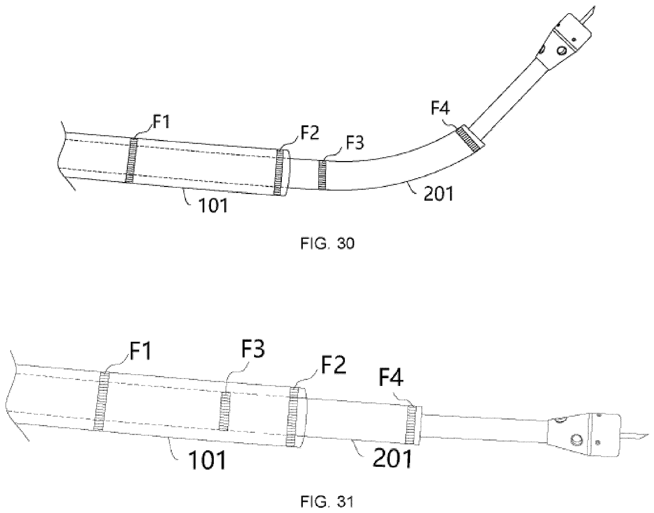

With reference to FIGS. 30 and 31, the flexible catheter 101 is provided with imaging marks F1 and F2. The distance between the imaging marks F1 and F2 on the flexible catheter 101 generally corresponds to the size of the straight segment 10132, for example, in the range of 40 to 80 mm.

Imaging marks F3 and F4 are provided on the bending sheath 201, and the distance between the imaging marks F3 and F4 on the bending sheath 201 is in the range of 40 to 80 mm. According to the imaging marks on the flexible catheter 101, the position of the straight segment 10132 and the distal end can be determined. According to the imaging marks on the bending sheath 201, the position of the distal end thereof can be determined, further, it can be determined whether the bending sheath 201 has extended out of the most distal end of the flexible catheter 101 by referring to the imaging mark on the most distal end of the flexible catheter 101. At least most of the bending sheath 201 should have extended out of the flexible catheter 101 to facilitate the bending. Otherwise, the flexible catheter 101 will interfere and affect the bending effect.

The technical solutions of the present disclosure are not limited to the technical solutions disclosed in the above embodiments, but further include technical solutions by combining the above technical features in any suitable manner. It should be noted that those skilled in the art can make several developments and modifications without departing from the principles of the present disclosure, which also fall into the protection scope of the present disclosure.

## Claims

1. An adaptive guiding device, comprising: a hollow flexible catheter, which is pre-shaped and has a bend in a natural state;
wherein a distal part of the flexible catheter comprises a distal section, the distal section comprises a transition segment and a straight segment, the transition segment is bendable so as to change an orientation of the straight segment, and a bending force application point is adjacent to a connection of the transition segment with the straight segment.

2. The adaptive guiding device of claim 1, wherein the straight segment has a length I, where 0 < I < 80 mm.

3. The adaptive guiding device of claim 2, wherein the length of the straight segment is in a range of 40 to 80 mm.

4. The adaptive guiding device of claim 1, wherein the transition segment is pre-shaped into an arc shape.

5. The adaptive guiding device of claim 4, wherein the transition segment has a central angle a5, and an arc bend of the transition segment has a radius R5, where 15° -< a5 -< 70°, and 25 mm ≤ R5 ≤ 45 mm.

6. The adaptive guiding device of claim 1, wherein the flexible catheter comprises a main section, a trans-aortic arch section and the distal section connected in sequence from proximal to distal and communicated with each other, and wherein the trans-aortic arch section has a curved structure simulating an aortic arch shape, and two ends of the transition segment are respectively tangent to the straight segment and the trans-aortic arch section.

7. The adaptive guiding device of claim 1, wherein the flexible catheter in the natural state is bent multiple times along its central axis toward different planes.

8. The adaptive guiding device of claim 7, wherein the flexible catheter is bent at least five times.

9. The adaptive guiding device of claim 8, wherein angles between planes of at least four consecutive bends are less than 90°.

10. The adaptive guiding device of claim 7, wherein an angle between two planes of one bend closest to a distal end is greater than 90°.

11. The adaptive guiding device of claim 6, wherein the trans-aortic arch section comprises a first arc segment, a second arc segment, a third arc segment and a fourth arc segment that are sequentially connected and tangent one after another, the first arc segment, the second arc segment, the third arc segment and the fourth arc segment are distributed on different planes, and wherein the first arc segment is connected and communicated with the main section, and the fourth arc segment is connected and communicated with the distal section.

12. The adaptive guiding device of claim 11, wherein the first arc segment has an angle a1, an arc bend of the first arc segment has a radius R1; the second arc segment has an angle a2, an arc bend of the second arc segment has a radius R2; the third arc segment has an angle a3, the arc bend of the third arc segment has a radius R3; and the fourth arc segment has an angle a4, and an arc bend of the fourth arc segment has a radius R4;
where 4.5° ≤ a1 ≤ 30°, 90 mm ≤ R1 ≤ 450 mm; 25° ≤ a2 ≤ 80°, 10 mm ≤ R2 ≤ 60 mm; 20° ≤ a3 ≤ 70°, 25 mm ≤ R3 ≤ 50 mm; 20° ≤ a4 ≤ 110 °, 20 mm ≤ R4 ≤ 50 mm.

13. The adaptive guiding device of claim 6, wherein angles between a central axis of a projection of the straight segment on a first plane, a second plane, and a third plane, respectively, and a central axis of a projection of the main section are α, β, and γ respectively, wherein the first plane is a vertical plane, the second plane is perpendicular plane perpendicular to the vertical plane, and the third plane is a horizontal plane perpendicular to both the vertical plane and the perpendicular plane, where 35° ≤ α ≤ 85°, 25° ≤ β ≤ 80°, and 20° ≤ γ ≤ 75°.

14. The adaptive guiding device of claim 13, wherein an angle between a central axis of a projection of the transition segment on the first plane and the central axis of the main section is δ, where 0 < δ ≤ 45°.

15. The adaptive guiding device of claim 6, wherein the adaptive guiding device further comprises an operating handle, and the operating handle is connected to the main section and is configured to drive the flexible catheter to move axially and circumferentially.

16. The adaptive guiding device of claim 15, wherein the adaptive guiding device further comprises a pull assembly, wherein the pull assembly comprises a pull wire, a guiding passageway and an anchor ring, the guiding passageway is defined in a wall of the flexible catheter for the pull wire to pass through, the anchor ring is fixedly connected to the transition segment of the distal section, and the pull wire is connected to the anchor ring and the operating handle respectively.

17. The adaptive guiding device of claim 16, wherein the anchor ring is adjacent to the connection of the transition segment with the straight segment.

18. The adaptive guiding device of claim 16, wherein the anchor ring also serves as an imaging mark.

19. The adaptive guiding device of claim 18, wherein a distal end of the straight segment is further provided with an imaging mark.

20. The adaptive guiding device of claim 16, wherein the pull wire is deflected around a central axis of the flexible catheter.

21. The adaptive guiding device of claim 20, wherein the pull wire is deflected by 90° around the central axis of the flexible catheter.

22. The adaptive guiding device of claim 16, wherein a start point of the guiding passageway at the distal section is A, point B is deflected by 90° relative to the point A in a circumferential direction of the flexible catheter, and a transitional guiding section is formed between point A and point B, wherein the transitional guiding section extends along a part or an entire of the trans-aortic arch section, or a part or an entire the main section.

23. The adaptive guiding device of claim 22, wherein the transitional guiding section has a length of L, where 0 mm < L ≤ 250 mm.

24. A transcatheter treatment system, comprising the adaptive guiding device of any one of claims 1 to 23 and a treatment device movably received in the flexible catheter, wherein the flexible catheter functions to establish a path from outside a human body to inside the human body, and the treatment device is configured to pass through an aortic arch via the flexible catheter.

25. The transcatheter treatment system of claim 24, wherein the treatment device is selected from at least one of a myocardial injection device, a myocardial ablation device, a valve repair device, or a valve replacement device.

26. The transcatheter treatment system of claim 25, wherein the treatment device comprises a bending sheath, an inner sheath and a treatment assembly, wherein the bending sheath is movably inserted in the flexible catheter and is bendable in a single direction, the inner sheath is movably inserted in the bending sheath, and the treatment assembly is inserted in the inner sheath.

27. The transcatheter treatment system of claim 26, wherein a bending direction of the bending sheath is different from a bending direction of the distal section of the flexible catheter of the adaptive guiding device.

28. The transcatheter treatment system of claim 27, wherein the bending direction of the bending sheath is substantially perpendicular to the bending direction of the distal section of the flexible catheter of the adaptive guiding device.

29. The transcatheter treatment system of claim 28, wherein a distal end of the inner sheath is fixedly connected with a limiting element, and after the inner sheath extends from a distal end of the bending sheath, the limiting element is configured to contact a tissue surface.

30. The transcatheter treatment system of claim 26, wherein the treatment assembly is selected from at least one of an injection assembly, an ablation assembly, a prosthetic heart valve, an annuloplasty ring, a valve clamping device, a suture, and a tissue anchor or a tissue puncture member.
